Europäisches Patentamt

**European Patent Office**

**Office européen des brevets**

(19)

(11) Veröffentlichungsnummer: **0 000 067**
**B1**

(12) **EUROPÄISCHE PATENTSCHRIFT**

(45) Veröffentlichungstag der Patentschrift:
**25.02.81**

(51) Int. Cl.³: **A 61 B 10/00, G 10 K 11/00**

(21) Anmeldenummer: **78100125.0**

(22) Anmeldetag: **12.06.78**

(54) Verfahren und Vorrichtung zur Ultraschalluntersuchung und Darstellung eines Objekts.

(30) Priorität: **13.06.77 US 806004**

(43) Veröffentlichungstag der Anmeldung:
**20.12.78 Patentblatt 78/1**

(45) Bekanntmachung des Hinweises auf die Patenterteilung:
**25.02.81 Patentblatt 81/8**

(84) Benannte Vertragsstaaten:
**BE CH DE FR GB NL SE**

(56) Entgegenhaltungen:
**DE-A-2 710 038**
**DE-B-2 534 974**
**FR-A-2 265 342**
**FR-A-2 269 074**
**SU-A-184 000**
**US-A-4 006 627**

(73) Patentinhaber: **NEW YORK INSTITUTE OF TECHNOLOGY, Wheatley Road, Old Westbury, New York 11 568 (US)**

(72) Erfinder: **Glenn, William E. jr. Dr., 650 Royal Plaza,, Fort Lauderdale Florida 33 301 (US)**

(74) Vertreter: **Daum, Martin, Dr. et al, Boehringer Mannheim GmbH. Sandhoferstrasse 116, D-6800 Mannheim 31 (DE)**

## Verfahren und Vorrichtung zur Ultraschalluntersuchung und Darstellung eines Objektes

Die Erfindung betrifft ein Verfahren zur Ultraschalluntersuchung und Darstellung eines Objektes auf einem Bildschirmgerät, bei welchem das Objekt mittels eines Ultraschallsender-/-empfängersystems und eines verschwenkbaren Abtastspiegels mit einem Ultraschallstrahl impulsweise in einer Schnittebene abgetastet wird, wobei die einer Schnittebene entsprechenden Ultraschallimpulse, die in dem Objekt annähernd äquidistante Strahlen bilden, in einem vom Zeilentakt des Bildschirmgerätes unterschiedlichen Zeittakt ausgelöst werden, sowie eine Vorrichtung zur Durchführung eines solchen Verfahrens.

Während der letzten zwei Jahrzehnte ist die Ultraschalltechnik in der klinischen Diagnostik stets bedeutungsvoller geworden. Die Ultraschalltechnik wurde unter anderem bereits im Bereich der Gynäkologie, der Neurologie und der Kardiologie verwendet, wobei sie z. B. bei der Sichtbarmachung subkutaner Blutgefäße (einschließlich kleinerer Gefäße) erfolgreich angewandt wurde.

Für die Anwendung der Ultraschalltechnik in der Medizin sprechen bedeutende Gründe: Ultraschall unterscheidet sich von anderer Art von Bestrahlung durch die damit verbundene harmlose Auswirkung auf lebende Systeme, weil sie rein mechanischer Wellennatur ist. Durch die Ultraschalltechnik ist Information erreichbar, die von anderen Methoden, beispeilsweise durch Untersuchung mit $\gamma$- und Röntgenstrahlen, nicht erreichbar ist. Vor allem ist das Risiko einer Verletzung bei der Verwendung von Ultraschall viel geringer als z. B. bei der Verwendung ionisierender Strahlen ($\gamma$- oder Röntgenstrahlen). Ultraschall wird in der Hauptsache als Pulsechomethode in der diagnostischen Technik verwendet, wozu Impulse von Ultraschallenergie periodisch von einem piezoelektrischen Geber, z. B. auf Blei-Zirkonat-Titanat Keramik-Basis, erzeugt werden. Jeder kleine Impuls Ultraschallenergie wird als Schallwelle gebündelt auf den Körper des Patienten gerichtet, wobei er über gegebenenfalls verschiedene Strukturen der Oberfläche eindringt. Hat eine Grenzfläche des Körpers eine Unregelmäßigkeit, an der sich die Phase der Ultraschallwelle ändert, so wird ein Teil der Ultraschallenergie wieder zurückgeworfen. Nach Abgabe eines Ultraschall-Impulses wird das Ultraschallgerät gewöhnlich auf Empfang gestellt, um reflektierte (oder Echo-)Signale vom Körper zurück in elektrische Signale wandeln zu können. Die Zeit, nach der diese Echosignale an den Empfänger zurückkommen, ist direkt vom Abstand der Reflexionsquelle und von der Schallgeschwindigkeit abhängig. Auch die Stärke des Schallechos ist interessant, weil sie Informationen über die Art einer Störstelle liefert.

Das Echo von Schallwellen kann auf verschiedene Weise dargestellt werden. Einerseits gibt es Geräte mit Verstärker, mit denen die dem empfangenen Ultraschallecho entsprechenden elektrischen Signale verstärkt an den vertikal ablenkenden Platten einer Kathodenstrahlröhre angelegt werden. Der Ausgang eines Zeitgenerators liegt dabei an der horizontalen Ablenkung der Kathodenstrahlröhre. Eine stetige Wiederholung des Impuls/Echo-Vorganges, synchronisiert mit dem Zeitgenerator führt dann zu einem stehenden Bild, sogenannte »A-Abtastung«, bei der die Zeit der Eindringtiefe proportional ist und vertikale Ablenkungen vorhandene Fehlordnungen signalisieren. Die Intensität dieser vertikalen Ablenkungen ist ein Maß für die Intensität des Echos.

Die Erfindung bezieht sich auf eine andere übliche Art bildlicher Darstellung von Ultraschallwellen, die sogenannte B-Abtastung, bei der die Echoinformation dem üblichen Fernsehbild entspricht, d. h. die empfangenen Schallechosignale werden zur Modulierung der Helligkeit des Schirmes je Abtaststelle verwendet. Dieser Bildschirmtyp wird speziell für durch den Körper gehende Schallwellenbetrachtung verwendet, so daß jede Intensitätsinformation mehrere Abtastlinien des Bildschirmes beansprucht und die aufeinanderfolgenden Positionen hintereinander werden zur Darstellung von aufeinanderfolgenden Linien auf dem Bildschirm verwendet. Mit dieser Technik wird ein Durchlichtbild in einer Ebene abgetastet und das resultierende Bild kann direkt betrachtet werden oder durch eine Photographie oder magnetisches Speicherband gelagert werden.

Gegenstand der Erfindung ist ein Verfahren und ein Gerät bei dem die durch den Körper gehende Ultraschallabtastung mit einem Reflektor durchgeführt wird, der mechanisch über einen bestimmten Winkel mit einer Frequenz, die mit der Bildfrequenz eines Bildschirmgerätes kompatibel ist, geführt wird. Der Reflektor müßte dazu möglichst sägezahnförmig entsprechend der Vertikalablenkung des Bildschirmes gedreht werden, um Verluste zu vermeiden. Das ist wegen der Trägheit der Spiegelmasse relativ zur Trägheit eines Elektronenstrahles aber praktisch nicht möglich. Ultraschallwellen sind also nicht ohne weiteres zur Darstellung auf einem elektrisch gesteuerten Bildschirm geeignet.

Es ist außerdem bekannt, daß Ultraschall am Grenzübergang zwischen einer Flüssigkeits- und einer Gasphase reflektiert. Dies hat zu einer Ultraschalltechnik geführt, bei der der Ultraschall durch eine abgeschlossene Flüssigkeit geleitet wird, in der er als Körperschall weniger Verluste hat. Wenn sich aber der reflektierende Abtastschirm in einer Flüssigkeit, wie Wasser befindet, stellt sich erst recht das Anpassungsproblem sehr schneller (Rück-)Bewegung — wegen großen inneren Widerstandes der Flüssigkeit nach einer langsamen Hinbewegung

— für Darstellung auf dem Bildschirm. Dies gilt insbesondere für Abtasteinrichtungen mit großem Abtastbereich, bei denen der innere Widerstand der Flüssigkeit einer Beschleunigung des Reflektionsschirmes entgegensteht.

Aus der SU-A-184 000 ist eine Vorrichtung zur Ultraschallabtastung eines Objektes bekannt, bei der ein Schallreflektor zur Ablenkung des von dem Schwingquarz kommenden Ultraschallstrahls in das Objekt benutzt wird. Auch hier befinden sich aus dem obengenannten Grund Quarz und Reflektor in einer Flüssigkeit. Der Schallreflektor kann nach einem gegebenen Programm mit Hilfe entsprechender Kurvenscheiben bewegt werden, um Objekte von komplizierter Form abzutesten. Die Probleme, die sich aus dieser möglicherweise nicht-linearen Bewegung des Reflektors für die Darstellung auf einem Bildschirm ergeben, wurden bei dieser bekannten Druckschrift nicht gelöst. Eine solche Bildschirmdarstellung ist auch gar nicht Gegenstand dieser Literaturstelle.

Die Aufgabe der Erfindung besteht nun darin, ein bildliches Darstellungssystem für Ultraschallwellenabtastung zu schaffen, bei dem auch dann eine verzerrungsfreie Darstellung auf einem Bildschirmgerät erreicht wird, wenn der zur Abtastung verwendete Ultraschallreflektor, den man kurz als Abtastspiegel bezeichnen kann, nicht der Vertikalablenkung des Bildschirmgerätes entsprechend bewegt wird und infolgedessen die Ultraschallimpulse in einem vom Zeilentakt des Bildschirmgerätes unterschiedlichen Zeittakt ausgelöst werden. Dabei soll insbesondere ein serienmäßiges Bildschirmgerät verwendbar sein, dessen Zeilentakt konstant ist, woraus sich eine erhebliche Kostenersparnis ergibt.

Diese Aufgabe wird bei einem Verfahren und einer Vorrichtung der eingangs bezeichneten Art gelöst durch die in den Ansprüchen gekennzeichneten Maßnahmen und Merkmale.

Ein Drehstellungsgeber ähnlich der erfindungsgemäß verwendeten Art ist aus der DE-B-25 34 974 bekannt. Dort wird er in einer Ultraschall-Abtastvorrichtung mit einem parabolischen Reflektor und mehreren mit einem gemeinsamen Kopf rotierenden Schwingquarzen verwendet, um eine Information über die Drehstellung des Kopfes zu gewinnen.

In bevorzugter Ausgestaltung der Erfindung wird der Reflektor gar nicht linear, sondern zum Beispiel sinusförmig, bewegt, wobei der Antrieb vorteilhaft mit einer Frequenz betrieben werden kann, die einer Resonanzfrequenz des Reflektors in der Flüssigkeit nahe kommt. So können unstete, plötzliche Bewegungen wie z. B. beim sägezahnförmigen Antrieb, vermieden werden. Die Genauigkeit eines Gerätes gemäß der Erfindung im überstrichenen Abtastbereich wird durch Messung, Speicherung und Anzeige der Schallwellen der Hin- und der Rückbewegung des Spiegels erhöht. Während der Abtastung in beiden Richtungen entsteht keine Totzeit. Bei einer weiteren bevorzugten Ausgestaltung der Erfindung werden die elektrischen Signale in Schieberegistern gespeichert. Die elektrischen Signale von der jeweiligen Abtastrichtung werden auf Speicherplätzen in der jeweiligen Richtung hintereinander gespeichert, wozu in besonders vorteilhafter Weiterentwicklung der Erfindung Schieberegister mit analogen Registern des CCD-Typs verwendet werden (CCD = Charge Couple Device = Ladungstransporteinrichtung, siehe US-A-3 882 271).

Weitere Ausgestaltungen der Erfindung werden in der folgenden Beschreibung an Hand von schematisch dargestellten Ausführungsbeispielen näher erläutert. Es zeigt

Fig. 1 einen Abtastkopf mit Empfänger beim Messen an einem Objekt,

Fig. 2 einen Querschnitt durch den Meßkopf und in blockartiger Darstellungsweise daran angeschaltete Bauelemente zur elektrischen Auswertung,

Fig. 3 ein Blockdiagramm einer Schaltung zur Erzeugung zeitkonstanter Signale, die in der Schaltung von Fig. 2 verwendet werden,

Fig. 4 ein Blockdiagramm des Speichersystems aus Fig. 2,

Fig. 5 bestehend aus den Unterteilen A—F zeigt den zeitlichen Ablauf von Impulsen während einer Periode zur Erläuterung der Arbeitsweise der Elektronik.

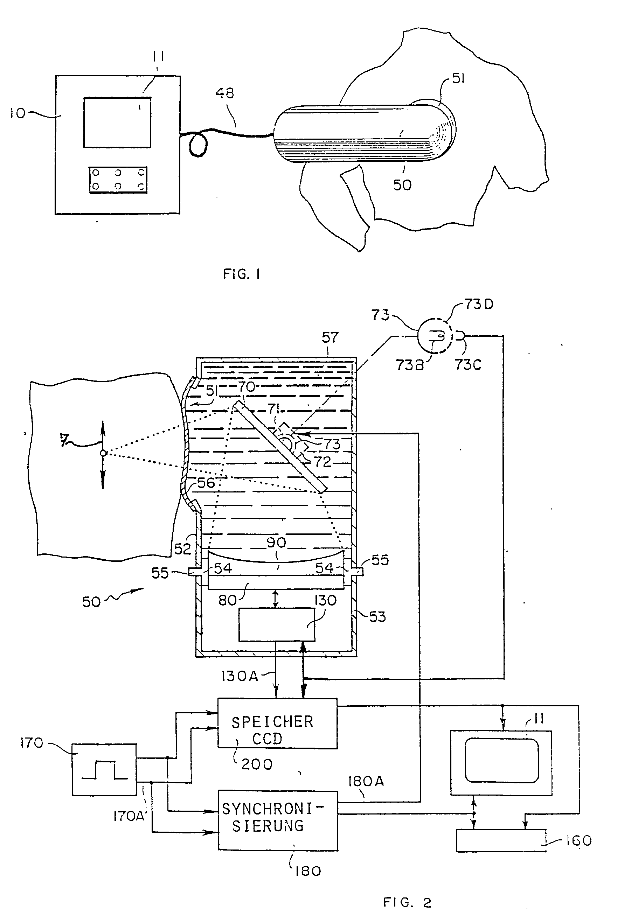

In Fig. 1 werden die äußeren Maße eines Abtastgerätes gemäß der Erfindung im Vergleich mit einem Objekt gezeigt. Das Kontrollpult 10 enthält einen Bildschirm 11, beispielsweise eine Kathodenstrahlröhre, in einer geeigneten Frontplatte. Außerdem können ein Videobandrecorder oder ein anderer Speicher z. B. auf der Basis photographischer Signale (Bildschirmkopierer), im Kontrollpult 10 enthalten sein, um die Signale zur Anzeige eines Bildes zu liefern. Ferner enthält das Kontrollpult 10 eine Energieversorgung und Teile der Schaltung für die Erzeugung zeitabhängiger Frequenz und zum Antrieb des Abtasters in dem Meßkopf 50. Der Meßkopf 50 (oder Sonde) ist mit dem Kontrollpult 10 mit einer elektrischen Leitung 48 verbunden. Der Meßkopf 50 des vorliegenden Ausführungsbeispiels ist im wesentlichen zylindrisch geformt und hat in der Nähe eines Endes ein Abtastfenster 51, das beispielsweise aus elastisch nachgiebigem Material Silicongummi besteht. Zur Handhabe des Gerätes wird der Meßkopf 50 in eine vom Bedienenden in der Hand zu haltenden Position gebracht, so daß das Abtastfenster 51 auf das abzutastende Objekt gerichtet ist. Bei dem in Fig. 1 dargestellten Objekt soll beispielsweise der Bereich um das Herz eines Menschen abgetastet werden. Selbstverständlich kann der Meßkopf auch zur Messung anderer Körperstellen oder anderer Objekte verwendet werden, auf die er mit Handgriff zu richten wäre.

Gemäß Fig. 2 wird der Meßkopf 50 im Querschnitt dargestellt, an den die zugehörigen Teile der Auswerteelektronik angeschlossen sind, die teils in der Sonde 50 und teils im Kontrollpult 10 angeordnet sein können. Das Gehäuse des Meßkopfes 50 schließt eine vordere Schalleitkammer 52, die eine Flüssigkeit enthält, und eine hintere Schallmeßkammer 53, die einen Teil der Elektronik enthält, ein. Beide Kammern 52 und 53 haben Zylinderform mit gleichgroßem Durchmesser, so daß sie mit Hilfe eines Rohres 54, das an seiner Außenseite einen ringförmigen Ansatz 55 besitzt, zu einem Zylinder zusammengesetzt werden können. Das (Innen-)-Rohr 54 trägt einen flächenförmigen Schallgeber/-empfänger 80, der im folgenden als Schallübertrager bezeichnet wird und eine Schallsammellinse 90, durch die die beiden Gehäuseteile voneinander getrennt werden (vgl. US-Patent 3 958 559). Das Abtastfenster 51 befindet sich am Ende der Kammer 52. Rings um die Fensteröffnung ist ein Ansatz vorgesehen, auf der eine elastisch nachgiebige Membran 56, beispielsweise Silicongummi-Membran, aufgezogen ist. Die vordere Schallkammer 52 ist mit einer Flüssigkeit 57, beispielsweise Wasser, ausgefüllt. Die Membran 56 soll so elastisch sein, daß sie sich mit dem Meßkopf an die Oberfläche des zu messenden Körpers glatt anlegt, um störende Reflektionen von Schallwellen an einem Übergang zwischen der Flüssigkeit des Gerätes zum Objekt möglichst gering zu halten.

Eine flächenhaft dargestellte schallreflektierende Abtasteinrichtung 70, im folgenden als Abtastspiegel bezeichnet, ist in der Flüssigkeit 57 zwischen der Schallinse 90 und dem Abtastfenster 51 angeordnet. Natürlich kann die Oberfläche des nicht gewölbt gezeichneten Abtastspiegels gebogen sein, um reflektierte Schallwellen zu fokussieren oder zu zersteuen. Der Abtastspiegel 70 ist an einer senkrecht zur Zeichenebene liegenden Achse 71 befestigt, die durch die Gehäusewand der vorderen Schalleitkammer 52 hindurchgeführt sein kann, um von außen mit einem kleinen elektrischen Motor 72, der die Hin- und Herbewegung erzeugt, betrieben zu werden. Ein ebenfalls auf der Drehachse des Schallspiegels und am Gehäuse 52 befestigter Drehstellungsgeber 73 ist in Fig. 2 noch besonders herausgezeichnet worden.

Der gestrichelt dargestellte Drehstellungsgeber 73 kann wie die äußeren Teile des Elektromotors in einem nichtdargestellten übergestülpten weiteren Gehäuseteil untergebracht sein. Der Schallübertrager 80 ist direkt mit einer Sende-/Empfangschaltung 130 verbunden, von der abwechselnd schallanregende Impulse ausgehen und vom Schallübertrager 80 zurück-kommende Schallechoimpulse empfangen wer-den. Es ist nicht dargestellt, daß zwischen dem Schallübertrager 80 und der Sende-/Empfangschaltung 130 noch verschiedene an sich bekannte elektrische Einrichtungen zur Konzentrierung des Ultraschallstrahles vorgesehen sein können.

Zur dynamischen Fokussierung beispielsweise mit Stufenlinsen wird auf das US-Patent No. 4 084 582 verwiesen.

Die Sende-/Empfangschaltung 130 enthält einen Vorverstärker und ist mit einem Speichersystem 200 über die elektrische Leitung 130 A verbunden. Das Speichersystem 200 liegt mit einem Ausgang am Eingang des Sichtgerätes 11 und des Aufnahmegerätes 160, die zur Darstellung und/oder zur Speicherung der anzuzeigenden Bilder vorgesehen sind. Eine Verstärkerregelung dazu ist in unserem US-Patent No. 4 043 181 beschrieben. Mit einer solchen Verstärkerregelung soll die Meßempfindlichkeit gesteuert werden.

Eine Zeittaktschaltung 170 liefert zeitkonstante Impulse zur Synchronisation des elektrischen Gerätes; die mit konstanter Frequenz abgegebenen elektrischen Signale werden an das Speichersystem 200 und außerdem an die Schaltung 180 zur Kontrolle des Abtastspiegels und der Ablenkung des Sichtgerätes gelegt. Diese Schaltung, die im folgenden Synchronisierschaltung genannt wird, liefert elektrische Signale zur Kontrolle der Abtastbewegung des Abtastspiegels 70 und auch der vertikalen und horizontalen Ablenkung der Röhre des Sichtgerätes, sowie des Aufnahmegerätes 160.

Das so beschriebene Gerät arbeitet in großen Zügen wie folgt: Der Impulsgenerator der Sende-/Empfangschaltung 130 erzeugt elektrische Impulse, die dem Schallübertrager 80 zugeleitet werden. Die dabei entstehenden akustischen Wellen werden durch die Linse 90 auf die Oberfläche des Abtastspiegels 70 gerichtet und von diesem in den zu beobachtenden Körper eingetragen. Die in Fig. 2 gepunktet dargestellten Linien zeigen den Bereich, in dem die Ultraschallwellen hauptsächlich verlaufen. Nachdem ein Wellenzug in den Körper eingedrungen ist, wird die Sende-/Empfangschaltung 130 auf Empfang geschaltet. In dieser Schaltstellung können reflektierte Schallwellen von dem Schallübertrager 80 über den Abtastspiegel 70 aus dem Objekt in elektrische Signale umgewandelt werden. Diese Signale gelangen über das Speichersystem 200 auf das Sichtgerät 11. Für einen flächenhaft darstellenden Bildschirm entspricht eine einer bestimmten Spiegelstellung entsprechende Abtastung einer horizontalen Linie des Bildschirmes. Unterschiedliche Echosignale entstehen bekanntlich an verschiedenen Grenzflächen im Körper. Die zweite Dimension des gewünschten Querschnittsbilds erhält man durch eine relativ langsame mechanische Schwenkbewegung des Abtastspiegels 70, wobei der Tastbereich in der Fig. 2 durch einen doppelseitig gerichteten Pfeil 7 dargestellt ist.

Taktimpulse $C_1$ aus dem Drehstellungsgeber kontrollieren die Sende-/Empfangschaltung 130 des Schallübertragers 80 mit einer solchen Frequenz, daß pro Zeile des Sichtgerätes 11 ein Schallimpuls erzeugt wird. Dabei wird berücksichtigt, daß Echo-Signale aus tieferliegenden Objektteilen wegen ihres längeren Weges und der relativ langsamen Schallgeschwindigkeit später zurückkommen.

Die Synchronisierschaltung 180 erzeugt Vertikal- und Horizontalablenkungssignale, die dem Sichtgerät 11 und dem Aufnahmegerät 160 zugeführt werden. Weiter erzeugt sie ein Motorantriebssignal, das über Leitung 180 A an den Motor 72 gelangt, der für die gewünschte Oszillationsbewegung des Abtastspiegels 70 sorgt. Wegen der Nachteile und Probleme für abrupte (unstetige) Bewegungen von Körpern in Flüssigkeiten wird der Abtastspiegel 70 nicht linear, sondern vorzugsweise sinusförmig (aber keinesfalls sägezahnförmig) bewegt. Mit der beschriebenen Elektronik wird aus der sinusförmigen Abtastbewegung ein sägezahnförmiges Signal zur Anzeige, wie erforderlich für Bildschirme, geschaffen. Dazu dienen auch die Signale des Drehstellungsgebers 73, rückgekoppelt über das Speichersystem 200.

Der Drehstellungsgeber ist mit einer feststehenden Lichtquelle 73 B und einem Photodetektor 73 C versehen, die an einem Blendenring 73 D anliegen, der Öffnungen in gleichen Abständen aufweist. Der Drehstellungsgeber wird gemeinsam mit dem Abtastspiegel 70 bewegt. Wegen der sinusförmigen Bewegung des Abtastspiegels 70 haben die vom Drehstellungsgeber 73 kommenden Impulse sinusförmig abwechselnd große oder kleine Abstände, nicht wie die gleichgroßen Abstände der Impulse der Zeittaktschaltung 170 (Fig. 5 E und 5 F).

Das Speichersystem 200 besteht vorteilhaft aus sogenannten CCD-Bausteinen (Charge Couple Device, siehe US-PS 3 882 271), in denen Ladungen auf hintereinanderliegenden Speicherplätzen verschoben werden.

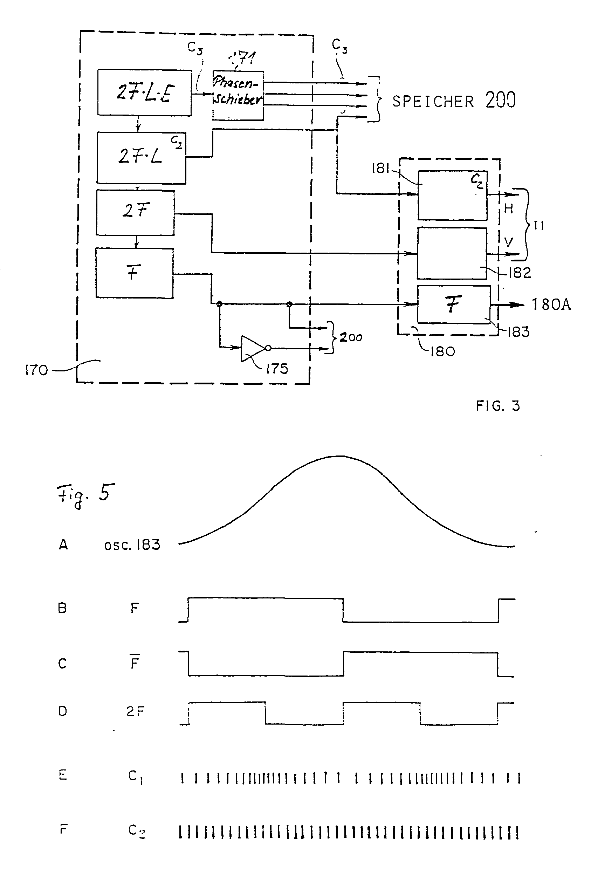

In Fig. 3 ist die Zeittaktschaltung im einzelnen dargestellt. Mit einer solchen Schaltung wird eine Bezugsgröße für den Speichervorgang geschaffen, um die mit ungleichmäßigen, bevorzugt sinusförmig sich ändernden Zeitabständen ankommenden Bildinformationen an die Wiedergabe durch das Sichtgerät 11 anzupassen, dessen Zeilenfrequenz und Bildfrequenz konstant sind.

Wie bekannt, entspricht die Information einer Zeile eines B-Abtastsystems der Lage der Störstellen in dem Objekt, die auf dem Weg der Schallwellen in den Körper gelegen haben.

Die Zeittaktschaltung 170 erzeugt ferner Taktsignale und zeitabhängige Signale, die mit der Synchronisierschaltung 180 und dem Speichersystem 200 verbunden sind. Einer von den zeitabhängigen Takten für die Synchronisierschaltung 180 und für das Speichersystem 200 ist das Taktsignal C 2, das auf der gleichen Frequenz wie die Zeilenablenkung für den Kathodenstrahloszillographen des Sichtgerätes 11 arbeitet. Die Leitung für diese Signale ist in Fig. 2 mit Bezugszeichen 170 A bezeichnet.

Die Signale für den Spiegelantrieb werden über die elektrische Leitung 180 A an den Elektromotor, der die entsprechende Abtastbewegung des Abtastspiegels 70 erzeugt, geleitet. Die Synchronisierschaltung 180 erzeugt außerdem die Signale, die zur Steuerung der vertikalen und horizontalen Ablenkung des daran angeschlossenen Bildschirms 11 und des Aufnahmegerätes 160 notwendig sind. Im vorliegenden Beispiel wird der Motor nicht-linear, vorzugsweise sinusförmig, also nicht wie gewöhnlich sägezahnförmig, (das der typischen Form der horizontalen Ablenkung für einen Bildschirm 11 entspricht) betrieben. Der Ausgang des Drehstellungsgebers 73 wird als Taktsignal C 1 bezeichnet, das aus einem Impulszug besteht, der einer Winkelbewegung des Abtastspiegels 70 entspricht.

Ein solches Signal wird beispielsweise, wie in Fig. 2 dargestellt, durch eine feststehende Lichtquelle 73 B mit einem Photoempfänger 73 C erzeugt, zwischen denen ein auf der Motorachse 71 befestigter Blendenring 73 D mit gleich großen hintereinanderliegenden gitterförmigen Blendenöffnungen vorgesehen ist. Während die Impulse C 2 des Taktgenerators gleichen zeitlichen Abstand haben, haben die Impulse C 1 vom Drehmomentgeber je nach Lage unterschiedlich große zeitliche Abstände zueinander, die von der Abtastgeschwindigkeit und der jeweiligen Lage des Abtastspiegels 70 abhängen. Wenn bei dem vorliegenden Ausführungsbeispiel der Abtastspiegel 70 sinusförmig bewegt wird, liegen die Impulse des Drehstellungsgebers C 1 besonders dicht zueinander, wenn der Abtastspiegel 70 durch die Mittellage schwingt. Die Impulse C 1 liegen besonders weit auseinander, wenn der Abtastspiegel an einer Endlage seine Bewegungsrichtung umkehrt. Die relative Lage der Impulse zueinander wird beispielsweise in Fig. 5 E dargestellt, die im weiteren noch genau beschrieben wird.

Bei der vorliegenden Ausführungsform werden die Taktimpulse C 1 vom Drehstellungsgeber, die mit der Lage des Abtastspiegels 70 synchronisiert sind, und somit der Richtung der eindringenden Schallwelle entsprechen, als Taktsignale der Echoinformation für das Speichersystem 200 verwendet. Das Speichersystem 200 besteht vorzugsweise aus einem Ladungstransportspeicher (sogenannter CCD-Baustein, siehe US-PS 3 882 271), vergleichbar mit einem Eimer-Ketten-Schieberegister. Die Information des Speichers 200 wird mit einem Takt C 2 wieder herausgelesen, die an die Zeilentaktfrequenz des Bildschirmes 11 angepaßt ist. Auf diese Weise kann der Abtastspiegel 70 in einer besonders wirksamen nichtlinearen Weise, beispielsweise in der Nähe einer natürlichen Resonanz betrieben werden und dabei genaue Informationen zur Anzeige auf einem gewöhnlichen Bildschirm liefern.

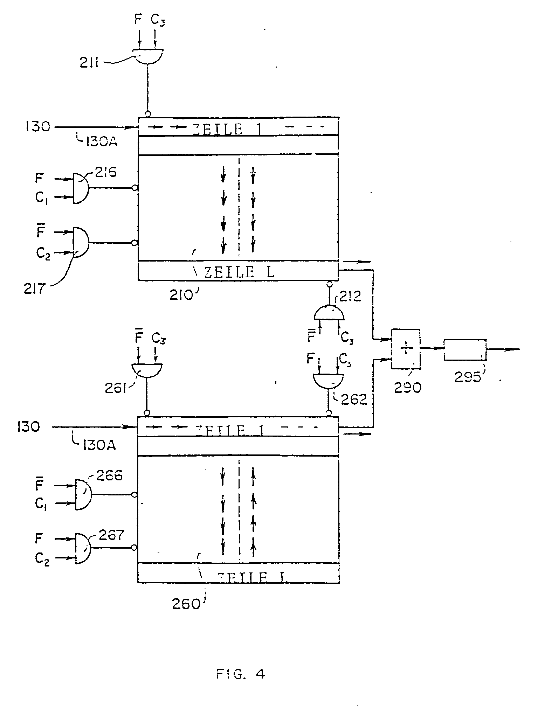

Die Zeittaktschaltung 170 und die Synchronisierschaltung 180 für den Spiegelantrieb 72 und den Bildschirm 11, der Informationen einer Abtastbewegung in beiden Richtungen erhält, ist in Fig. 3 ausführlicher dargestellt. Bei einer solchen Einrichtung wird das Tastverhältnis dadurch erhöht, daß keine Totzeiten entstehen und weil der Abtastspiegel 70 stetig bewegt werden kann. Im einzelnen wird dies durch ein Paar von Speicherregister 210 und 260 im Speichersystem 200 (Fig. 2) erreicht, die im weiteren Text im Zusammenhang mit Fig. 4 beschrieben sind. Eines dieser Speicherregister erhält neue Informationen von dem Abtastspiegel 70 durch eine Abtastbewegung in einer Richtung, während die in der vorangegangenen Abtastbewegung gewonnene Information aus dem anderen Speicherregister ausgelesen und auf das Sichtgerät 11 gegeben wird. Das Umgekehrte geschieht bei der nächsten zurückgehenden Abtastbewegung, so daß in dem Speichersystem stets gleichzeitig Informationen ein- und ausgelesen werden.

In Fig. 3 ist die Bildfrequenz des Bildschirmes 11 mit F bezeichnet, während mit einer Rate von 2 F gespeichert wird. L ist die Zeilenzahl und E die Anzahl der helligkeitssteuerbaren Punkte einer Zeile eines Fernsehbildes. Außerdem kann die Frequenz F, die der Abtastbewegung des Abtastspiegels 70 entspricht, an eine Resonanzfrequenz des Spiegels in der Flüssigkeit angepaßt werden, in der der Spiegel gelagert ist. Diese Frequenz F kann beispielsweise 15 Herz betragen und L und E können an die Zeilenzahlen normaler Fernsehgeräte angepaßt werden. Im Beispiel wurden 250 Zeilen eines aus 500 Zeilen bestehenden Feldes, bei dem je 1 Zeile übersprungen wurde, mit 500 Elementen pro Zeile verwendet, so daß L 250 und E 500 betragen.

Die dem System zugrunde liegende Taktfrequenz $C\,3$ erzeugt Impulse mit einer Frequenz: $2 \times F \times L \times E$. Wie im folgenden beschrieben wird, bestimmt diese Frequenz den Basistakt der Speichervorgänge. Mit dem Phasenschieber 171 werden drei um 120° zueinander versetzte Phasenimpulse $C\,3$ erzeugt. Ferner ist ein Taktgenerator vorgesehen, der mit einer Frequenz $2 \times F \times L$ Impulse $C\,2$ erzeugt, die durch eine Division der Frequenz von $C\,3$ durch E ermittelt werden. Außerdem wird eine Taktfrequenz $2\,F$ durch Division der Taktfrequenz $2 \times F \times L$ (d. h. von $C\,2$) durch L erzeugt. Eine weitere Taktfrequenz, bezeichnet mit F, wird durch Division dieser Taktfrequenz durch 2 erhalten.

Wie bereits beschrieben, wird mit der Taktfrequenz $C\,2$, die der Zeilenfrequenz entspricht, das zeilenweise Auslesen des Speichersystems 200 gesteuert. Weiter wird sie zur Synchronisation der horizontalen Abtastlinien des Sichtgerätes 11 verwendet. Die zuletzt genannte Synchronisation wird durch Kopplung an den horizontalen Ablenkungsschaltkreis 181 (Signal H) erreicht, der ein Teil der Synchronisierschaltung 180 ist. Mit der Frequenz 2 F wird der Bildschirm durch eine Kopplung an die Vertikalablenkungsschaltung 182 (Signal V) in der Synchronisierschaltung 180 verbunden. Der Ausgang der Schaltsysteme 181 und 182, der mit normalen Ablenkungsgeneratoren verbunden ist, liefert die Signale für die normale horizontale und vertikale Ablenkung des Elektronenstrahls eines Sichtgerätes 11. Mit der Taktfrequenz F wird der Oszillator 183 synchronisiert, der sinusförmige Signale für den Antrieb des Ablenkspiegels 70 auf der Frequenz F, wie in Fig. 5 A dargestellt, liefert, mit denen der Antriebsmotor 72 versorgt wird. Die Taktimpulse mit der Frequenz F sind ferner an einen Inverter 175 gekoppelt, so daß Impulse mit der Frequenz $\bar{F}$ erzeugt werden, die auch an das Speichersystem 200 gegeben werden. Die Taktimpulse mit der Frequenz $\bar{F}$ unterscheiden sich von den Taktimpulsen mit der Frequenz F durch entgegengesetzt liegende Phasenlage.

Fig. 4 zeigt eine besondere Ausführungsform des Speichersystems 200 aus Fig. 2. Ein Paar von Speicherregistern 210 und 260 in der Art von Dreiphasenladungsschiebespeichern (sogenannte CCD) sind vorgesehen, die als solche in der US-PS 3 882 271 beschrieben sind. In der zitierten Patentschrift wird beschrieben, wie ein einem Videosignal entsprechendes elektrisches Signal diskriminiert und in einem Ladungsschiebespeicherregister zeilenweise aufgehoben wird, um die Information einer Zeile vollständig in einen Speicher zu geben, und daß die Information einer Zeile abgegeben werden kann, während Informationen für die nächstfolgende Zeile aufgenommen werden. Selbstverständlich ist ein Gerät der Erfindung auch mit anderen käuflich erwerbbaren Speichern zu betreiben.

Zur besseren Erläuterung des Speichers 210 sind mit Pfeilen dargestellte Informationen in Zeile 1 gezeichnet, die unter Taktkontrolle der Frequenz $C\,3$ stehen, wie bisher üblich und bekannt ist. Nachdem eine Zeile voll gespeichert ist, wird mit Taktfrequenz $C\,1$ die nächste Zeile aufgeladen, wobei die Taktfrequenz $C\,1$ vom Drehstellungsgeber entnommen wird (Fig. 2). Der Speicher wird in der Weise gefüllt, daß die Information vorangegangener Zeilen zeilenweise weitergeschoben wird. Auf diese Weise enthält die letzte Zeile des Speichers die erste Zeile der eingelesenen Information. Der Speicher wird vorzugsweise auf die Weise wieder ausgelesen, daß die zuerst eingegebene Information zuerst wieder ausgegeben wird.

Die Information von dem Speicher soll aber in einer anderen als der eingelesenen Frequenz $C\,1$, nämlich mit der der Zeilenfrequenz entsprechenden Frequenz $C\,2$ ausgelesen werden. Da keine Informationen in dieser Frequenz ankommen, wird der Speicher ohne Wiederauffüllen geleert, aber die gleichzeitig ankommenden Informationen in der Taktfrequenz $C\,1$ auf einen zweiten Speicher 260 gegeben. Zur Darstellung auf dem Bildschirm werden dann die Speicher wechselweise ausgelesen, wozu logische Bausteine und ein Additionsglied 290 vorgesehen sind. Ferner ist noch ein Filter 295 vorgesehen, mit dem die der Information aufgeprägten Taktimpulse wieder entfernt werden. Das Gerät der Erfindung könnte natürlich auch mit einem Paar anders aufgebauter »Eimerkettenschieberegister« geschaltet sein, wenn der Speicher 200 wie folgt arbeitet:

Beim Abtasten in einer Richtung wird zunächst ein Speicher 210 in der beschriebenen Weise beladen. Während der Rückbewegung des Reflektors 70 in entgegengesetzter Richtung wird die gespeicherte Information sägezahnförmig für den Bildschirm geeignet ausgelesen, während neu eingehende Informationen auf den Speicher 260 gegeben werden, usw. Weil die Informationen von beiden Abtastrichtungen zu einem gemeinsamen Bildschirmsignal kombiniert werden, und weil der Abtaststrahl in beiden Richtungen empfangen wird, können laufend Informationen entgegengenommen und dargestellt werden, weil entweder Speicher 210 oder Speicher 260 empfangsbereit und entweder Speicher 260 oder Speicher 210 zur Abgabe von Signalen für den Bildschirm 11 bereit sind.

Die Bereitschaft der Speicher 210 oder 260 wird von der jeweiligen Phasenlage des jeweiligen Taktsignals F oder $\bar{F}$ gesteuert.

Das Eintakten der Information in den Speicher 210 wird durch ein UND-Gatter 211 kontrolliert, der den in 3 Phasen aufgeteilten Taktimpuls C 3 mit dem Speicher 210 bei positiver Phasenlage der Taktfrequenz F verbindet. Zum Herauslesen jeder Abtastzeile ist ein weiteres UND-Gatter 212 vorgesehen, das Informationen nur dann durchläßt, wenn das in der Phase entgegengesetzte Taktsignal $\bar{F}$ positiv ist. Einlesen und Auslesen im Speicher 260 verläuft während entgegengesetzter Phasen der Taktfrequenzen F und $\bar{F}$. Während nämlich das UND-Gatter 261 Einlesen nur bei positivem Phasenverlauf des Signals $\bar{F}$ erlaubt, wird von dem UND-Gatter 262 das Auslesen gesteuert, das nur bei positivem Verlauf des Taktsignals F möglich ist.

Der Zeilenvorschub wird beim Einlesen im Speicher 210 durch das UND-Gatter 216 mit einer Frequenz C 1 gesteuert, während der Zeilenvorschub beim Auslesen nur mit einer Frequenz C 2 über das UND-Gatter 217 möglich ist. Entsprechende logische Bausteine, UND-Gatter 266 und 267 sind für das Ein- und das Auslesen am Speicher 260 vorgesehen.

Zum besseren Verständnis der Arbeitsweise eines Gerätes der Erfindung sind in Fig. 5 Impulsdiagramme dargestellt, die die verschiedenen verwendeten Impulsreihen in Abhängigkeit von der Zeit zeigen. Fig. 5 A zeigt einen sinusförmigen Spannungsverlauf, der zum Antrieb des Abtastspiegels 70 dem Oszillator 183 entnommen wird. Dieser sinusförmigen Steuerfrequenz entspricht die Bildfrequenz F. Während des Anstieges der sinusförmigen Spannung vom Oszillator tastet der Spiegel 70 beispielsweise von der linken bis zur rechten Seite den Meßbereich ab, während gleichzeitig Schallwellen in das Objekt abgegeben werden. Während des Spannungsabfalls der sinusförmigen Frequenz wird der Abtastspiegel entsprechend von rechts nach links zurückbewegt. Damit wird der Abtastspiegel 70 wieder in Ausgangsstellung zurückgestellt. In den Fig. 5 B und 5 C sind volle Durchläufe der Taktsignale F und $\bar{F}$ dargestellt. In Fig. 5 D ist die Taktfrequenz 2 F dargestellt, aus der die vertikale Ablenkfrequenz V für die Bildschirmanzeige durch die Ablenkeinheit 182 (Fig. 3) gewonnen wird. Fig. 5 E zeigt den typischen Verlauf der Taktsignale C 1, die von dem Drehstellungsgeber 73 ausgehen und Fig. 5 F zeigt schematisch einen Impulszug der Taktsignale, wie sie in Fig. 3 dargestellt, entstehen. Die Anzahl und Intensität der in den Fig. 5 E und 5 F in Form von vertikalen Linien dargestellten Taktimpulse wäre bei natürlichem Maßstab größer und dichter, so daß in dem Gezeigten nur ungefähre Verhältnisse sichtbar werden. Dabei soll deutlich werden, daß die Takte C 1 von dem Drehstellungsgeber 73 im Bereich der Wendepunkte der Bewegung des Abtastspiegels weiter auseinander liegen, als in dem Bereich der Bewegung des Abtastspiegels, in dem er mit großer Geschwindigkeit von rechts nach links oder von links nach rechts durch die Mitte eines Abtastweges schwingt (wenn die Sinuswelle ihre maximale Steigung hat). Die Takte C 2 von der Zeittaktschaltung 170 sind zeitkonstant, wie die Takte C 3, erhalten durch Division — gezeigt in Fig. 3. Während einer ersten Halbwelle der Frequenz F, während der das Gatter 216 geöffnet ist, wird die Information mit der Taktfrequenz C 1 in den Speicher 210 eingegeben und die Taktfrequenz C 2 wird dabei zum Auslesen der Information aus dem Speicher 260 der von dem Gatter 267 geöffnet ist, verwendet. Während der nächsten Halbwelle der Frequenz F, während der die invertierte Frequenz $\bar{F}$ positiv ist, wird mit der Taktfrequenz C 1 vom Drehstellungsgeber der Speicher 260 zeilenweise aufgeladen, weil das Gatter 266 bei positivem $\bar{F}$ geöffnet ist, und dabei wird die Taktfrequenz C 2 nunmehr zum Auslesen der Information aus dem Speicher 210, der bei invertierter Frequenz $\bar{F}$ durch Gatter 217 geöffnet ist, verwendet. Die Mechanik des Antriebs für den Abtastspiegel 70 kann zur Verbesserung der mechanischen Eigenschaften eines Gerätes der Erfindung noch ausgestaltet werden. Zwecks weiterer Anpassung sollten am besten L Blendenöffnungen in dem Blendenring 73 D des Drehstellungsgebers 73 vorgesehen sein, so daß die Zahl der Impulse C 1 während eines Durchgangs gleich der Anzahl der zeitabhängigen Taktimpulse C 2 ist.

## Patentansprüche

1. Verfahren zur Ultraschalluntersuchung und Darstellung eines Objektes auf einem Bildschirmgerät (11), bei welchem das Objekt mittels eines Ultraschallsender-/-empfängersystems (80, 130) und eines verschwenkbaren Abtastspiegels (70) mit einem Ultraschallstrahl impulsweise in einer Schnittebene abgetastet wird, wobei die einer Schnittebene entsprechenden Ultraschallimpulse, die in dem Objekt annähernd äquidistante Strahlen bilden, in einem vom Zeilentakt des Bildschirmgerätes (11) unterschiedlichen Zeittakt ausgelöst werden, dadurch gekennzeichnet, daß die pro Schnittebene empfangenen Echosignale zeilenweise in ein Speichersystem (200) eingelesen, mit einer der Zeilenfrequenz des Bildschirmgerätes (11) angepaßten Frequenz zeilenweise aus dem Speichersystem (200) ausgelesen und zur Helligkeitssteuerung der Bildpunkte des Bildschirmgerätes (11) verwendet werden.

2. Verfahren nach Anspruch 1, dadurch gekennzeichnet, daß der Abtastspiegel (70) mit sich ändernder Winkelgeschwindigkeit bewegt wird.

3. Verfahren nach Anspruch 2, dadurch gekennzeichnet, daß der Abtastspiegel (70) mit sich sinusförmig ändernder Winkelgeschwindigkeit bewegt wird.

4. Vorrichtung zur Ultraschall-Untersuchung und Darstellung eines Objektes auf einem Bildschirmgerät (11) mit einem Ultraschallsender-/-empfängersystem (80, 130) und einem im Wege des Ultraschallstrahles angeordneten, über einen Antrieb (72) verschwenkbaren Abtastspiegel (70), wobei die einer Schnittebene entsprechende Ultraschallimpulse, die in dem Objekt annähernd äquidistante Strahlen bilden, in einem vom Zeilentakt des Bildschirmgerätes (11) unterschiedlichen Zeittakt ausgelöst werden, dadurch gekennzeichnet, daß die Vorrichtung folgende Einrichtungen aufweist: einen Drehstellungsgeber (73) für die Angabe der den Strahlrichtungen der Ultraschallimpulse jeweils zugeordneten Winkelstellungen des Abtastspiegels (70), der eine der Durchlaufgeschwindigkeit des Abtastspiegels (70) durch die Winkelstellungen entsprechende, zur Steuerung des Ultraschallsender-/-empfängersystems (80, 130) verwendete erste Impulsfolge (C₁) erzeugt, ein Speichersystem (200), in welchem die echorepräsentativen Signale mit einem der ersten Impulsfolge (C₁) entsprechenden ersten Zeittakt zeilenweise abgespeichert werden, und eine Zeittaktschaltung (170) zur Erzeugung eines der Zeilenfrequenz des Bildschirms (11) entsprechenden zweiten Zeittaktes (C₂), der sowohl zum zeilenweisen Auslesen der Signale aus dem Speichersystem (200), als auch zum Eingeben der ausgelesenen Signale in das Bildschirmgerät (11) und/oder in den Speicher eines Aufnahmegerätes (160) dient.

5. Vorrichtung nach Anspruch 4, dadurch gekennzeichnet, daß der Antrieb (72) für den Abtastspiegel (70) für eine sich ändernde Winkelgeschwindigkeit ausgelegt ist.

6. Vorrichtung nach Anspruch 5, dadurch gekennzeichnet, daß der Antrieb (72) für den Abtastspiegel (70) für eine sich sinusförmig ändernde Winkelgeschwindigkeit ausgelegt ist.

7. Vorrichtung nach einem der Ansprüche 4 bis 6, dadurch gekennzeichnet, daß das Speichersystem (200) zwei Speicherregister (210, 260) einschließt, die Zeittaktschaltung (170) einen Zeittakt (C₃) mit der Frequenz 2 FLE erzeugt, wobei F die Frequenz der Spiegelschwenkbewegung, L die Zeilenzahl und E die Anzahl der helligkeitssteuerbaren Punkte einer Zeile des Bildschirmgerätes (11) sind, und ferner die Zeilenzahl L mit der Zahl der vom Drehstellungsgeber (73) während einer Halbschwingung des Abtastspiegels (70) erzeugten Impulse übereinstimmt, und daß eine aus logischen Bauelementen (211, 212, 216, 217, 261, 262, 266, 267) aufgebaute Schaltung zur Auswertung der echorepräsentativen Signale vorgesehen ist, derart, daß während der einen Hälfte der Bewegung des Abtastspiegels (70) die echorepräsentativen Signale auf das eine Speicherregister (210) gegeben werden, während die bei der vorhergehenden Hälfte der Bewegung des Abtastspiegels (70) gespeicherten echorepräsentativen Signale dem anderen Speicherregister (260) entnommen werden, und daß das zeilenweise Auslesen der Signale aus dem einen Speicherregister (210) in der Reihenfolge deren Eingabe, aus dem anderen Speicherregister (260) in der deren Eingabe entgegengesetzten Reihenfolge erfolgt.

8. Vorrichtung nach Anspruch 7, dadurch gekennzeichnet, daß die Speicherregister (210, 260) vom ladungsgekoppelten (CCD) Typ sind.

9. Vorrichtung nach einem der Ansprüche 4 bis 8, dadurch gekennzeichnet, daß der Drehstellungsgeber (73) aus einer Lichtquelle (73 B), einem Fotoempfänger (73 C) und einem Blendenring (73 D) besteht.

**Claims**

1. Method for the ultrasonic investigation and representation of an object on an image display device (11), in which by means of an ultrasonic transmitter/receiver system (80, 130) and a displaceable scanning mirror (70) the object is scanned in a pulsed manner in a section plane by an ultrasonic beam, the ultrasonic pulses corresponding to a section plane, which form approximately equidistant rays in the object, being triggered at a timing different from the line scan timing of the image display device (11), characterised in that the echo signals received per section plane are read line-wise into a storage system (200), and read out from the storage system (200) with a line frequency suited to the line scan frequency of the image display device (11) and are used for brightness control of the image points of the image display device (11).

2. Method according to claim 1, characterised in that the scanning mirror (70) is moved with a changing angular speed.

3. Method according to claim 2, characterised in that the scanning mirror (70) is moved with a sinusoidally changing angular speed.

4. Device for the ultrasonic investigation and representation of an object on an image display device (11), with an ultrasonic transmitter/receiver system (80, 130) and a scanning mirror (70) displaceable by a drive (72) and arranged in the path of the ultrasonic beam, the ultrasonic pulses corresponding to a section plane, which form approximately equidistant rays in the object, being triggered at a timing different from the line scan timing of the image display device (11), characterised in that the device comprises the following arrangements: a rotary position transmitter (73) for giving the angular position of the scanning mirror associated with the beam direction of the ultrasonic pulse, which produces a first pulse train (C 1) corresponding to the traversing speed of the scanning mirror, used for controlling the ultrasonic transmitter/receiver system (80, 130), a storage system (200) in which the echo-representative signals are stored in a line-wise manner with a first line timing corresponding to the first pulse train C 1, and a timing circuit (170) for generating a second timing signal train (C 2), which serves for the line-wise reading of signals from the storage system (200) as well as for feeding the read out signals into the image display device (11) and/or into the store of a receiving device (160).

5. Device according to claim 4, characterised in that the drive (72) for the scanning mirror (70) is arranged for a varying angular speed.

6. Device according to claim 5, characterised in that the drive (72) for the scanning mirror (70) is arranged for a sinusoidally varying angular speed.

7. Device according to one of claims 4 to 6, characterised in that the storage system (200) includes two storage registers (210, 260), the timing circuit (170) produces a timing signal train (C 3) with the frequency 2 FLE, where F is the frequency of the oscillating movement of the mirror, L is the number of lines and E is the number of intensity controllable points of one line of the image display device (11), and furthermore the line number L coincides with the number pulses produced by the rotary position transmitter (73) during a half oscillation of the scanning mirror (70), and that a circuit constructed from logical components (211, 212, 216, 217, 261, 262, 266, 267) is provided for evaluating the echo representative signals, such that during one half of the movement of the scanning mirror (70) the echo representative signals are fed to one storage register (210), while in the preceding half of the movement of the scanning mirror (70) they are taken from the other storage register (260), the line-wise read out of the signals from the one storage register (210) being performed in the same sequence as their input, and from the other storage register (260) in a sequence opposite to their input sequence.

8. Device according to claim 7, characterised in that the storage register (210, 260) is of the charge coupled (CCD) type.

9. Device according to one of claims 4 to 8, characterised in that the rotary position transmitter (73) consists of a light source (73 B), a photo-receiver (73 C) and a masking ring (73 D).

## Revendications

1. Procédé pour l'examen par ultrasons et pour la représentation d'un objet sur un appareil de visualisation dans lequel on balaye l'objet au moyen d'un ensemble émetteur ultrasonique/récepteur ultrasonique (80, 130) et d'un miroir oscillant (70), par impulsions avec un faisceau ultrasonique dans un plan de section dans lequel les impulsions ultrasoniques correspondant à un plan de section et formant dans l'objet des faisceaux à peu près équidistants, sont déclenchées avec une fréquence de répétition différente de la période des lignes de l'appareil de visualisation, caractérisé en ce que les signaux d'écho reçus par plan de section sont introduits ligne par ligne dans un système à mémoire (200), puis lus ligne par ligne à partir de cette mémoire (200) avec une fréquence adaptée à celle des lignes de l'appareil de visualisation (11) et utilisés pour la commande de la luminosité des points d'image de l'appareil de visualisation (11).

2. Procédé selon la revendication 1, caractérisé en ce que le miroir de balayage (70) est actionné avec une vitesse angulaire variable.

3. Procédé selon la revendication 2, caractérisé en ce que le miroir de balayage (70) est actionné avec une vitesse angulaire qui varie selon une sinusolde.

4. Appareil pour l'examen par ultrasons et pour la représentation d'un objet sur un appareil de visualisation (11) au moyen d'un ensemble émetteur ultrasonique/récepteur ultrasonique (80,130) et d'un miroir de balayage oscillant (70) disposé dans le parcours du faisceau ultrasonique actionné par un mécanisme d'entrainement (72) dans lequel les impulsions ultrasoniques correspondant à un plan de section et formant dans l'objet des rayons à peu près équidistants, sont déclenchées avec une récurrence différente de la fréquence de la base de temps de l'appareil de visualisation (11), caractérisé en ce que l'appareil comprend les dispositifs suivants: un transmetteur de position angulaire (73) pour indiquer les positions d'angle du miroir de balayage (70) correspondant chaque fois aux directions des faisceaux ultrasoniques, qui engendre une première série d'impulsions ($C_1$) correspondant à la vitesse avec laquelle le miroir de balayage (70) parcourt les différentes positions d'angle, cette série d'impulsions ($C_1$) étant utilisée pour commander l'ensemble émetteur ultrasonique/récepteur ultrasonique (80, 130); un système à mémoire (200) dans lequel sont stockés ligne par ligne les signaux représentant l'écho, avec une première fréquence de répétition correspondant à la première série d'impulsions ($C_1$); et un circuit d'horloge (170) pour la création d'une seconde fréquence de répétition ($C_2$) qui est utilisée d'une part pour la lecture ligne par ligne des signaux stockés dans la mémoire (200), et d'autre part pour l'introduction des signaux lus dans l'appareil de visualisation (11) et/ou dans la mémoire d'un appareil d'entregistrement (160).

5. Appareil selon la revendication 4, caractérisé en ce que le mécanisme d'entrainement (72) du miroir de balayage (70) est prévu pour donner à ce dernier une vitesse angulaire variable.

6. Appareil selon la revendication 5, caractérisé en ce que le mécanisme d'entrainement (72) du miroir de balayage (70) est prévu pour donner à ce dernier une vitesse angulaire variable selon une sinusolde.

7. Appareil solon l'une quel conque des revendications 4 à 6, caractérisé en ce que le systeme à mémoire (200) comprend deux registres à mémoire (210, 260), en ce que le circuit d'horloge (170) engendre un signal périodique ($C_3$) de la fréquence 2 FLE, où F est la fréquence du mouvement oscillant du miroir, L le nombre de lignes et E le nombre de points à luminosité contrôlée d'une ligne de l'appareil de visualisation (11), le nombre de lignes L correspondant en outre au nombre des impulsions engendré pendant une demi-oscillation du miroir de balayage (70) par le transmetteur de position angulaire (73), et en ce qu'il est prévu un circuit composé d'éléments constitutifs logiques (211, 212, 216, 217, 261, 262, 266, 267) pour évaluer les signaux représentatifs de l'écho, de telle manière, que pendant l'une des moitiés du mouvement du miroir de balayage (70) les signaux représentatifs de l'écho soient introduits dans l'un des registres à mémoire (210) et qu'en même temps les signaux représentatifs de l'écho enregistrés pendant la moitié précédente du mouvement du miroir de balayage (70) soient retirés de l'autre des registres à mémoire (260) et qu'enfin la lecture ligne par ligne des signaux s'effectue à partir de l'un des registres à mémoire (210) dans l'ordre de leur entrée, et à partir de l'autre des registres à mémoire (260) dans l'ordre contraire de leur entrée.

8. Appareil selon la revendication 7, caractérisé en ce que les registres à mémoire (210, 260) sont du type à charge couplée (CCD).

9. Appareil selon l'une quelconque des revendications 4 à 8, caractérisé en ce que le transmetteur de position angulaire (73) comprend une source lumineuse (73 B), un récepteur photosensible (73 C et un réglage du diaphragme 73 (D).

FIG. I

FIG. 2

FIG. 3

Fig. 5

| A | osc. 183 | |
| B | F | |
| C | $\bar{F}$ | |
| D | 2F | |
| E | $C_1$ | |
| F | $C_2$ | |

13

FIG. 4